# EUROPEAN PATENT APPLICATION

(11) **EP 4 195 669 A1**
(43) Date of publication of application: **14.06.2023**
(21) Application number: 22159472.4
(22) Date of filing: 01.03.2022
(51) Int. Cl.: H04N 21/2187, H04N 21/4223, H04N 21/44, H04N 21/4402, H04N 21/472, G16H 40/67, A61B 5/00

(54) **IMAGE QUALITY MANAGEMENT SYSTEM**

(30) Priority: 13.12.2021 US 202163288677 P
(71) Applicant: Koninklijke Philips N.V., 5656 AG Eindhoven (NL)
(72) Inventor: KOKER, Ekin, Eindhoven (NL); STAROBINETS, Olga, Eindhoven (NL); DALAL, Sandeep Madhukar, Eindhoven (NL); LIU, Aaron, Eindhoven (NL)
(74) Representative: Philips Intellectual Property & Standards

(57) **Abstract**

A non-transitory computer readable medium (26s) stores instructions executable by at least one electronic processor (14s) to perform a method (100) of assisting a local operator (LO) during a medical procedure performed using a medical device (2). The method includes acquiring a video (17) of at least one component of the medical device, wherein the video comprises a time sequence of image frames; as the video is acquired, transmitting the acquired video to a remote electronic processing device (12) operable by a remote expert (RE); as the video is acquired, determining a state of the medical procedure as a function of time from the acquired video; and as the video is acquired, adjusting a data stream transmission setting of the transmitting as a function of time based on the determined state of the medical procedure as a function of time.

## Description

### FIELD OF THE INVENTION

The following relates generally to the remote imaging assistance arts, remote imaging examination monitoring arts, remote imaging examination data transmission arts, and related arts.

### BACKGROUND OF THE INVENTION

Radiology operations command centers (ROCC) are a promising way for large imaging centers or integrated delivery networks (IDNs) with diverse pools of technologists to share technologist expertise across an entire imaging network. By providing means of communication and remote console sharing, ROCC systems can empower the most experienced technologists to provide guidance and oversight for junior techs when performing difficult scans or facing challenges during exam acquisition.

For a remote expert to offer meaningful input and guidance, ROCC systems provides access to the imaging console screen and scanner room video feeds and displays real-time updates based on the information extracted from streaming video sources. These data streams should be of high resolution to ensure the remote expert has sufficient information upon which to base provided assistance. However, there are high bandwidth requirements and thus costs associated with streaming high quality (high resolution, high frame rate) data.

The following discloses certain improvements to overcome these problems and others.

### SUMMARY OF THE INVENTION

In one aspect, a non-transitory computer readable medium stores instructions executable by at least one electronic processor to perform a method of assisting a local operator during a medical procedure performed using a medical device. The method includes acquiring a video of at least one component of the medical device, wherein the video comprises a time sequence of image frames; as the video is acquired, transmitting the acquired video to a remote electronic processing device operable by a remote expert; as the video is acquired, determining a state of the medical procedure as a function of time from the acquired video; and as the video is acquired, adjusting a data stream transmission setting of the transmitting as a function of time based on the determined state of the medical procedure as a function of time.

In another aspect, a non-transitory computer readable medium stores instructions executable by at least one electronic processor to perform a method of assisting a local operator during a medical procedure performed using a medical device. The method includes transmitting an acquired video of at least one component of the medical device to a remote electronic processing device operable by a remote expert, wherein the video comprises a time sequence of image frames; determining a state of the medical procedure as a function of time from the acquired video; and adjusting a data stream transmission setting of the transmitting as a function of time based on the determined state of the medical procedure as a function of time.

In another aspect, a method of assisting a local operator during a medical procedure performed using a medical device includes transmitting an acquired video of at least one component of the medical device to a remote electronic processing device operable by a remote expert, wherein the video comprises a time sequence of image frames; determining a state of the medical procedure as a function of time from the acquired video; adjusting a data stream transmission setting of the transmitting as a function of time based on the determined state of the medical procedure as a function of time; automatically detecting two-way communication between the local operator and the remote expert; and adjusting the data stream transmission setting of the transmitting as a function of time further based on the automatic detection of the two-way communication.

One advantage resides in dynamically balancing between providing a remote expert with high quality data versus controlling bandwidth usage by adjusting video data transmission settings between electronic devices operated by a local technician and a remote expert based on the state of the medical procedure.

Another advantage resides in ensuring reliable transmission of video data between electronic devices operated by a local technician and a remote expert during a medical procedure.

Another advantage resides in reduced errors, repeats, and reduced wasted time for medical procedures.

Another advantage resides in determining a state of a medical procedure performed using a medical device before adjusting video data transmission settings between electronic devices operated by a local technician and a remote expert during the medical procedure.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 diagrammatically shows an illustrative apparatus for determining data transmission settings in accordance with the present disclosure.

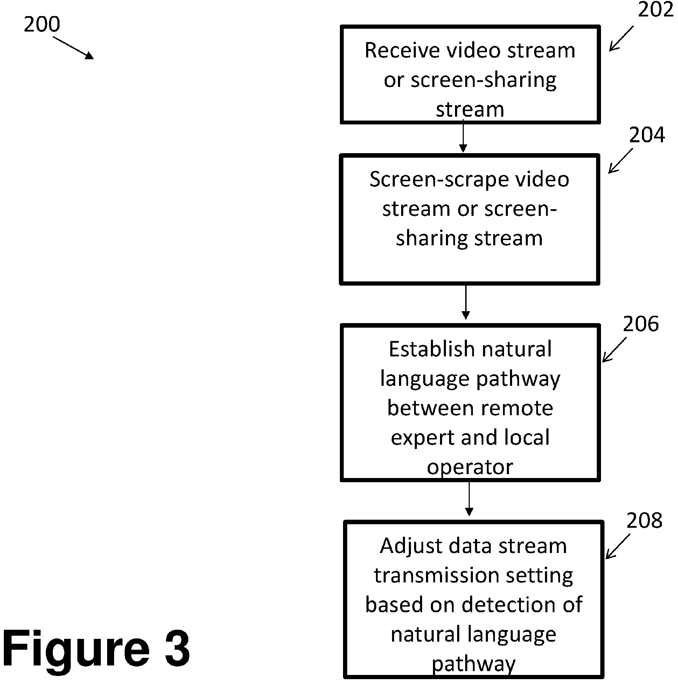

Figs. 2 and 3 show example flow charts of operations suitably performed by the apparatus of Fig. 1.

### DETAILED DESCRIPTION OF EMBODIMENTS

The following relates a system for providing real-time remote expert assistance to imaging technicians during imaging procedures. More particularly, the following relates to controlling bandwidth of a console feed (or console stream) and bay camera data streams transmitted from each imaging bay to the monitoring remote expert.

To this end, frames of the console feed video are analyzed to determine a scanner state in (near) real time. The state may for example be an idle state between examinations, a protocol prescription state, an active scanning state, or so forth. For each state, a corresponding set of data stream transmission settings are used. To reduce bandwidth in less critical phases (e.g., idle), the transmitted frame rate can be reduced and/or the image resolution (and hence file or packet size) of each frame can be reduced. In a variant approach, the resolution can be kept the same, but frame data transmission size reduced by employing lossy data compression on a per-frame or data stream basis, thus reducing the amount of data transmitted at potentially some loss in image fidelity.

While in the illustrative embodiments the state of the medical procedure is determined from the scraped controller display feed, a similar approach can be applied for the bay camera video feed. In this case, the states might be, for example, patient in imaging bay and not in scanner, or otherwise. The scraped controller display and the scraped bay camera video (if available) can be combined to provide more information from which to determine the state of the medical procedure. For example, knowing both the controller state based on the scraped controller display and the state of patient presence/absence/preparation based on the bay camera video provides more information for assessing the state of the medical procedure since it may not be possible, for example, to assess whether the patient is being loaded into an imaging scanner based only on the scraped controller display. The state can also be determined based on *a priori* knowledge of the imaging protocol. For example, when a change from a "State A" to a "State B" is detected from the video, then if it is known from the imaging protocol being performed that "State B" will last for 10 minutes (for example) and that the video quality can be reduced during "State B", then the video quality can be reduced for the next 10 minutes.

In another variant embodiment, a state of the medical imaging device can be determined from the medical imaging protocol based on information on that protocol execution provided by the imaging device controller itself, if there is a data feed from the controller providing this information. For example, there could be large time segments during image acquisition that no interaction between a local operator performing the medical imaging procedure, and a remote expert monitoring the medical imaging procedure would be needed or expected. That is, the medical imaging procedure set-up, a beginning of the medical imaging procedure protocol, transition to another medical imaging procedure protocol, an end of medical imaging procedure have the greatest likelihood of interactions. However, there could also be specific points during the image acquisition that requires review or interaction from the monitoring remote expert. These points can be designated and determined by the screen scraping (i.e. what appears on the screen) or the system can send a signal to a remote monitoring device, or the local operator can create the indication.

In another variant embodiment, the expert user interface (UI) could have a button or other user control for transmitting full resolution data streams when the user control is selected. This could be useful, for example, to give the expert maximum information while actively assisting the imaging technician. In a further variant, if the videoconferencing or other active assistance communication path is tied to the screen scraping and bay camera feeds then the active assistance can be automatically detected, and the full resolution data streams supplied.

In illustrative embodiments, a structural similarity index metric is used for assessing scanner state, but a wide range of approaches could be used (e.g. detecting text in the scraped controller frames indicative of scanner state).

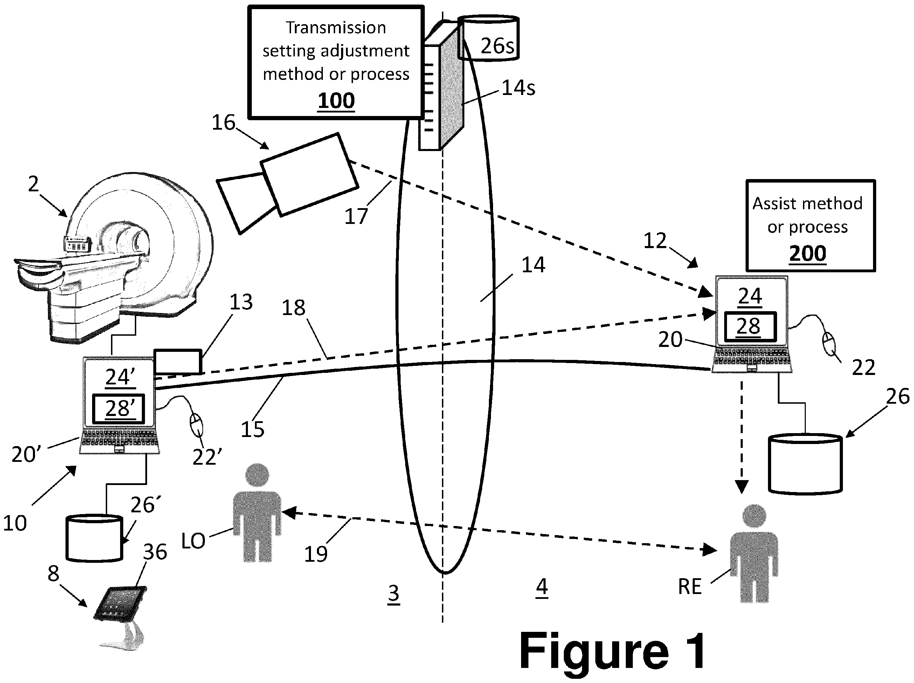

With reference to Fig. 1, an apparatus for providing assistance from a remote medical imaging expert RE to a local radiologist technician or local technician operator LO is shown. Such a system is also referred to herein as a radiology operations command center (ROCC). As shown in Fig. 1, the local operator LO, who operates a medical imaging device (also referred to as an image acquisition device, imaging device, and so forth) 2, is located in a medical imaging device bay 3, and the remote expert RE is disposed in a remote service location or center 4. It should be noted that the remote expert RE may not necessarily directly operate the medical imaging device 2, but rather provides assistance to the local operator LO in the form of advice, guidance, instructions, or the like.

The image acquisition device 2 can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device 2 may also be a hybrid imaging device such as a PET/CT or SPECT/CT imaging system. While a single image acquisition device 2 is shown by way of illustration in Fig. 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center 4 may provide service to multiple hospitals. The local operator LO controls the medical imaging device 2 via an imaging device controller 10. The remote expert RE is stationed at a remote workstation 12 (or, more generally, an electronic controller 12).

The imaging device controller 10 includes an electronic processor 20', at least one user input device such as a mouse 22', a keyboard, and/or so forth, and a display device 24'. The imaging device controller 10 presents a device controller graphical user interface (GUI) 28' on the display 24' of the imaging device controller 10, via which the local operator LO accesses device controller GUI screens for entering the imaging examination information such as the name of the local operator LO, the name of the patient and other relevant patient information (e.g. gender, age, etc.) and for controlling the (typically robotic) patient support to load the patient into the bore or imaging examination region of the imaging device 2, selecting and configuring the imaging sequence(s) to be performed, acquiring preview scans to verify positioning of the patient, executing the selected and configured imaging sequences to acquire clinical images, display the acquired clinical images for review, and ultimately store the final clinical images to a Picture Archiving and Communication System (PACS) or other imaging examinations database. In addition, while Fig. 1 shows a single medical imaging device bay 3, it will be appreciated that the remote service center 4 (and more particularly the remote workstation 12) is in communication with multiple medical bays via a communication link 14, which typically comprises the Internet augmented by local area networks at the remote operator RE and local operator LO ends for electronic data communications. Consequently, the remote operator RE is susceptible to unplanned interruptions as the imaging technologists working in these numerous imaging bays may experience unexpected difficulties and call for assistance from the remote operator RE.

As diagrammatically shown in Fig. 1, in some embodiments, a camera 16 (e.g., a video camera) is arranged to acquire a video stream 17 of a portion of the medical imaging device bay 3 that includes at least the area of the imaging device 2 where the local operator LO interacts with the patient, and optionally may further include the imaging device controller 10. The video stream 17 is sent to the remote workstation 12 via the communication link 14, e.g., as a streaming video feed received via a secure Internet link.

In other embodiments, the live video feed 17 of the display 24' of the imaging device controller 10 is, in the illustrative embodiment, provided by a video cable splitter 15 (e.g., a DVI splitter, a HDMI splitter, and so forth). In other embodiments, the live video feed 17 may be provided by a video cable connecting an auxiliary video output (e.g. aux vid out) port of the imaging device controller 10 to the remote workstation 12 of the operated by the remote expert RE. Alternatively, a screen mirroring data stream 18 is generated by screen sharing software 13 running on the imaging device controller 10 which captures a real-time copy of the display 24' of the imaging device controller 10, and this copy is sent from the imaging device controller 10 to the remote workstation 12. These are merely nonlimiting illustrative examples.

The communication link 14 also provides a natural language communication pathway 19 for verbal and/or textual communication between the local operator LO and the remote expert RE, in order to enable the latter to assist the former in performing the imaging examination. For example, the natural language communication link 19 may be a Voice-Over-Internet-Protocol (VOIP) telephonic connection, a videoconferencing service, an online video chat link, a computerized instant messaging service, or so forth. Alternatively, the natural language communication pathway 19 may be provided by a dedicated communication link that is separate from the communication link 14 providing the data communications 17, 18, e.g., the natural language communication pathway 19 may be provided via a landline telephone. These are again merely nonlimiting illustrative examples.

Fig. 1 also shows, in the remote service center 4 including the remote workstation 12, such as an electronic processing device, a workstation computer, or more generally a computer, which is operatively connected to receive and present the video 17 of the medical imaging device bay 3 from the camera 16 and to present the screen mirroring data stream 18 as a mirrored screen. Additionally or alternatively, the remote workstation 12 can be embodied as a server computer or a plurality of server computers, e.g., interconnected to form a server cluster, cloud computing resource, or so forth. The workstation 12 includes typical components, such as an electronic processor 20 (e.g., a microprocessor), at least one user input device (e.g., a mouse, a keyboard, a trackball, and/or the like) 22, and at least one display device 24 (e.g., an LCD display, plasma display, cathode ray tube display, and/or so forth). In some embodiments, the display device 24 can be a separate component from the workstation 12. The electronic processor 20 is operatively connected with a one or more non-transitory storage media 26. The non-transitory storage media 26 may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation 12, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a graphical user interface (GUI) 28 for display on the remote operator display device 24.

The medical imaging device controller 10 in the medical imaging device bay 3 also includes similar components as the remote workstation 12 disposed in the remote service center 4. Except as otherwise indicated herein, features of the medical imaging device controller 10 disposed in the medical imaging device bay 3 similar to those of the remote workstation 12 disposed in the remote service center 4 have a common reference number followed by a "prime" symbol (e.g., processor 20', display 24', GUI 28') as already described. In particular, the medical imaging device controller 10 is configured to display the imaging device controller GUI 28' on a display device or controller display 24' that presents information pertaining to the control of the medical imaging device 2 as already described, such as imaging acquisition monitoring information, presentation of acquired medical images, and so forth. The real-time copy of the display 24' of the controller 10 provided by the video cable splitter 15 or the screen mirroring data stream 18 carries the content presented on the display device 24' of the medical imaging device controller 10. The communication link 14 allows for screen sharing from the display device 24' in the medical imaging device bay 3 to the display device 24 in the remote service center 4. The GUI 28' includes one or more dialog screens, including, for example, an examination/scan selection dialog screen, a scan settings dialog screen, an acquisition monitoring dialog screen, among others. The GUI 28' can be included in the video feed 17 or provided by the video cable splitter 15 or by the mirroring data stream 17' and displayed on the remote workstation display 24 at the remote location 4.

Fig. 1 shows an illustrative local operator LO, and an illustrative remote expert RE. However, the ROCC optionally provides a staff of remote experts who are available to assist local operators LO at different hospitals, radiology labs, or the like. The ROCC may be housed in a single physical location or may be geographically distributed. The server computer 14s is operatively connected with a one or more non-transitory storage media 26s. The non-transitory storage media 26s may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer 14s, various combinations thereof, or so forth. It is to be understood that any reference to a non-transitory medium or media 26s herein is to be broadly construed as encompassing a single medium or multiple media of the same or different types. Likewise, the server computer 14s may be embodied as a single electronic processor or as two or more electronic processors. The non-transitory storage media 26s stores instructions executable by the server computer 14s. In addition, the non-transitory computer readable medium 26s (or another database) stores data related to a set of remote experts RE and/or a set of local operators LO. The remote expert data can include, for example, skill set data, work experience data, data related to ability to work on multi-vendor modalities, data related to experience with the local operator LO and so forth.

Furthermore, as disclosed herein the server 14s implements an electronic system that performs a method or process 100 of adjusting a transmission setting of the video 17 between the remote workstation 12 and the ROCC device 8. It will be appreciated that where the server computer 14s is providing simultaneous ROCC support for a substantial number of imaging bays, the transmission of the controller displays and optionally other data streams such as bay camera video can take up a large amount of bandwidth. However, usual approaches for moderating bandwidth such as using lower resolution, reducing frame rate, and/or employing lossy compression are problematic in the ROCC context since the remote expert RE may require high resolution data upon which to base medically related advice. In embodiments disclosed herein, these competing considerations are balanced by automatically determining the state of the medical imaging procedure in real time based on the data stream(s) (e.g., the scraped controller display) and adjusting settings of the video transmission to reduce bandwidth during portions of the procedure that are unlikely to be analyzed in detail by the remote expert RE, while adjusting the settings to maximize video quality (e.g. maximum resolution and frame rate, with reduced or eliminated lossy compression) during portions of the procedure that are likely to be analyzed in detail by the remote expert RE.

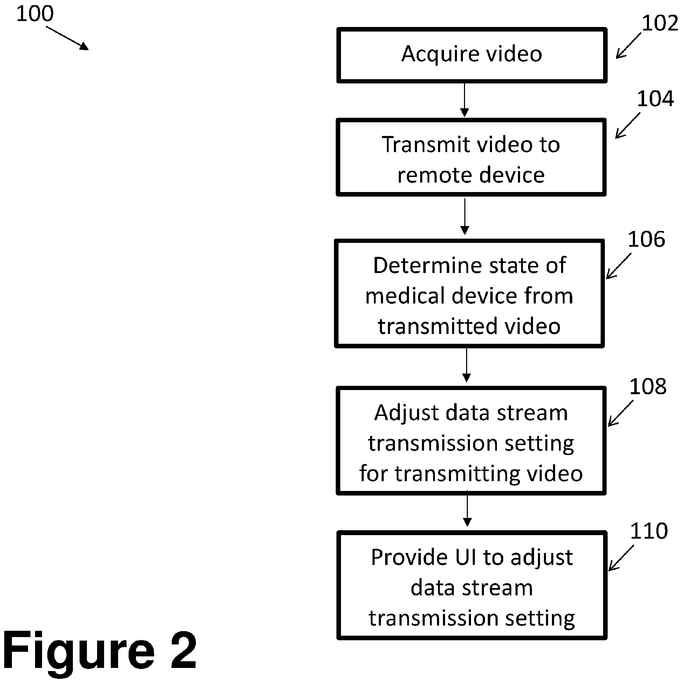

With reference to Fig. 2, and with continuing reference to Fig. 1, an illustrative embodiment of the method 100 is diagrammatically shown as a flowchart. At an operation 102, the video feed 17 of at least one component of the medical device 2 is acquired as a time sequence of image frames. The video feed 17 comprises a series of screen captures from the medical imaging device controller 10. In one embodiment, the acquiring operation 102 includes screen-scraping a display of the medical device 2 (i.e., the medical imaging device controller 10). The time sequence of image frames comprises a time sequence of screen-scraped images of the medical imaging device controller 10. In another embodiment, the acquiring operation 102 includes acquiring the video stream 17 using the video camera 16, which can be arranged to image a display of the medical device 2 (i.e., the medical imaging device controller 10). The time sequence of image frames comprises a time sequence of images of the medical imaging device controller 10. At an operation 104, during the acquiring of the video feed 17, the acquired video feed 17 is transmitted to the remote workstation 12 operable by the remote expert RE from either the camera 16 or the ROCC device 8.

At an operation 106, during the acquiring of the video feed 17, a state of the medical procedure is determined as a function of time from the acquired video feed 17. For example, when the video feed 17 comprises video of the medical imaging device controller 10, the states of the medical procedure can include one of an idle state, a protocol prescription state, and an active scanning state of the medical imaging device 2. In some embodiments, a structural similarity index metric (stored in the non-transitory computer readable medium 26 of the remote workstation 12, or in the non-transitory computer readable medium 26s of the server computer 14s) can be used to determine the state of the medical procedure. In another example, text screen-scraped from the acquired video feed 17 can be used to determine the state of the medical procedure. In a variant thereof, if there is *a priori* information about the imaging protocol, such as duration of certain states of the imaging protocol, this can be used as well, e.g. detection of a switch to a state that is known to last for 10 minutes can be used to determine the state for the next 10 minutes.

In another example, the state determination operation can include determining the stated of the medical imaging device 2 from a medical imaging protocol. For example, there could be large time segments during image acquisition that no interaction between the local operator LO performing the medical imaging procedure, and the remote expert RE would be needed or expected. That is, the medical imaging procedure set-up, a beginning of the medical imaging procedure protocol, transition to another medical imaging procedure protocol, an end of medical imaging procedure have the greatest likelihood of interactions. However, there could also be specific points during the image acquisition that requires review or interaction from the remote expert RE. These points can be designated and determined by the screen scraping (i.e. what appears on the screen) video 17, or the imaging device 2 can send a signal to the remote workstation 12, or the local operator LO can create an indication that attention is required using the ROCC device 8.

At an operation 108, during the acquiring of the video feed 17, a data stream transmission setting of the transmitting from the camera 16 or the ROCC device 8 to the remote workstation 12 can be adjusted based on the determined state of the medical procedure as a function of time. In one example, the adjusting operation 108 includes adjusting a transmitted frame rate of the acquired video 17 transmitted to the remote workstation 12. In another example, the adjusting operation 108 includes adjusting a resolution of the image frames of the acquired video 17 transmitted to the remote workstation 12. In another example, the adjusting operation 108 includes adjusting a compression parameter of a data compression process applied to the acquired video 17 before transmission to the remote workstation 12. The adjustments made in operation 108 are designed to maximize video quality of the video feed 17 (at the cost of higher bandwidth) during portions of the medical procedure that may be analyzed in detail by the remote expert RE, while minimizing bandwidth (at the cost of lower video quality) during portions of the medical procedure that are unlikely to be analyzed in detail by the remote expert RE. For example, the remote expert RE is unlikely to be analyzing the video feed of the controller display in detail while the patient is being loaded into the imaging device, and hence the video feed of the controller display can be at a lower resolution and/or lower frame rate during this state of the procedure in order to reduce bandwidth. Conversely, if a bay camera video feed is also being provided to the remote expert RE then this may be switched to maximum resolution during patient loading so the remote expert has a detailed view of any issues that may arise during the patient loading. During another state of the procedures, such as execution of an imaging scan, the situation may be reversed: a high resolution feed of the controller display may be desired while the feed from the bay camera (if available) can be switched to a low resolution and/or low frame rate.

At an optional operation 110, the UI 28 can be provided on the display device 24 of the remote workstation 12 to include a user control button selectable by the remote expert RE to adjust the data stream transmission setting of the transmitting. For example, the UI 28 can optionally provide a "full resolution" button or the like via which the remote expert RE can switch a video feed to full resolution. This can be useful if, for example, the automated procedure state-based settings are for a low resolution because the remote expert is deemed unlikely to be examining the video feed in detail, but the remote expert to the contrary does want to examine the video feed in detail. By selecting the "full resolution" button the remote expert is thereby provided with the full resolution feed (i.e., full frame pixel resolution and fastest frame rate).

Referring back to Fig. 1, when the local operator LO requires assistance from the remote expert RE, the communication link 14 connects the local operator LO/ remote expert RE. The GUI 28 is provided as a remote assistance UI on the display device 24 operable by a remote expert RE. The UI 28 provides two-way communication between the local operator LO and the remote expert RE via which the remote expert can provide assistance to the local medical imaging device operator LO.

The remote workstation 12 of the selected remote expert RE, and/or the medical imaging device controller 10 being run by the local operator LO, is configured to perform a method or process 200 for providing assistance from the remote expert RE to the local operator LO. For brevity, the method 200 will be described as being performed by the remote workstation 12. The non-transitory storage medium 26 stores instructions which are readable and executable by the at least one electronic processor 20 (of the workstation 12, as shown, and/or the electronic processor or processors of a server or servers on a local area network or the Internet) to perform disclosed operations including performing the method or process 200.

A suitable implementation of the assistance method or process 200 is as follows. The method 200 is performed over the course of (at least a portion of) an imaging procedure performed using the medical imaging device 2, and the remote expert RE is one selected via the method 100 having availability. To perform the method 200, at an operation 202, the workstation 12 in the remote location 4 is programmed to receive at least one of: (i) the video 17 from the video camera 16 of the medical imaging device 2 located in the medical imaging device bay 3; and/or (ii) the screen sharing 18 from the screen sharing software 13; and/or (iii) the video 17 tapped by the video cable splitter 15. The video feed 17 and/or the screen sharing 18 can be displayed at the remote workstation display 24, typically in separate windows of the GUI 28. At an operation 204, the video feed 17 and/or the screen sharing 18 can be screen-scraped to determine information related to the medical imaging examination (e.g., modality, vendor, anatomy to be imaged, cause of issue to be resolved, and so forth). In particular, the GUI 28 presented on the display 24 of the remote workstation 12 preferably includes a window presenting the video 17, and a window presenting the mirrored screen of the medical imaging device controller 10 constructed from the screen mirroring data stream 18, and status information on the medical imaging examination that is maintained at least in part using the screen-scraped information. This allows the remote operator RE to be aware of the content of the display of the medical imaging device controller 10 (via the shared screen) and also to be aware of the physical situation, e.g., position of the patient in the medical imaging device 2 (via the video 17), and to additionally be aware of the status of the imaging examination as summarized by the status information. During an imaging procedure, at an operation 206, the natural language communication pathway 19 is established between the remote medical professional workstation 12 and the ROCC device 8, and is suitably used to allow the local operator LO and the remote operator RE to discuss the procedure and in particular to allow the remote operator to provide advice to the local operator LO.

At an operation 208, once the natural language communication pathway 19 is detected between the data stream transmission setting of the transmitting can be adjusted as a function of time further based on the automatic detection of the two-way communication. Typically, the operation 208 will increase video feed resolution to maximum frame pixels and maximum frame rate. In a variant embodiment, if the remote expert RE is assigned to handle multiple imaging examinations simultaneously, then when the operation 206 is executed to establish a natural language pathway between the remote expert and the local operator of any one of those multiple imaging examinations then the resolution of the video feed(s) of that examination are maximized (at the cost of higher bandwidth) and the resolution of the video feed(s) of the other examinations being handled by the remote expert are lowered (thereby reducing bandwidth consumed by those other examinations the remote expert is not presently actively assisting).

### EXAMPLE

The following describes another example of the method 100. The disclosed ROCC system is a highly secure collaboration platform that enables virtualized imaging operations. To provide the remote experts RE with real-time scan-in progress updates, create alerts, evaluate the quality of acquired images, etc., the ROCC system processes console screens and in-scanner video footage for pertinent information. However, processing and analysing every frame from every console screen and/or scanner room video is a resource prohibitive task. Therefore, it is important to find ways of identifying appropriate image quality management strategies such as 1) when to speed up/ slow down rate at which console screen data or the medical imaging bay 3 video feed 17 are analysed, 2) when low resolution data is acceptable versus instances when high-fidelity image data is required (i.e., the medical imaging bay 3 is empty versus patient position and coaching are taking place).

The state determination operation 106 includes an algorithm (implemented in the server computer 14s or the remote workstation 12) that determines the stage (i.e. state) of the medical imaging examination (i.e., medical procedure) based on the information extracted from the console screen page of the medical imaging device controller 10 or the medical imaging bay 3 video feed 17. For console screens, this can be accomplished by matching the console screen page to a repository of scanner-specific templates using similarity metrics such as structural similarity index (SSIM), zero norm, Manhattan norm, etc. or a combination of multiple metrics.

A repository of page templates (stored in the server computer 14s) could be matched against console screen frames to determine the page in use. Alternatively, certain elements (e.g., pop-up windows, button colors, sequence names, and so forth) found on specific pages could be extracted and used to determine the stage of the medical imaging examination.

To get the state of the medical imaging examination, an algorithm can be run constantly processing video content with sufficiently high framerate to capture all the critical moments. Keeping computation cost low is essential. This can be achieved by reducing the size or the resolution of the image. The focus can be only on the critical areas of the image; for instance, detecting changes related to buttons. In this way, the size of input image is reduced. Furthermore, a variable-resolution-wavelet-like-template can also be used; the streaming video 17 can be compared with the template globally but at a low resolution, while certain critical areas of the video can be compared with the template at a higher resolution. In this way, the resolution of the image used by the monitoring algorithm can be reduced.

Similarly, video feed streamed from within the medical imaging device bay 3 could be used to identify the step in the scanning workflow. Based on individuals present in the scanning room, their positions, equipment in use - it is possible to deduce the stage in the scan progression. For instance, a local operator LO and a patient getting situated would signal that the medical imaging examination is just starting. A patient inside the medical device 2 would indicate active scanning in progress. Additionally, console-screen extracted information may resolve any ambiguities associated with video feed data alone. Information regarding the stage of the medical imaging examination could then be used to determine the way in which data should be processed for console extraction, as well as video feed processing.

Once the state of the medical imaging examination is known, this information can be combined with other factors to determine the minimal image quality required for console screen and/or scanner video processing. For instance, a patient information page could be used to extract elements regarding the patient or the protocol in use. However, once these elements are extracted, the ROCC system can sample frames at lower rate or use lower resolution images. On the other hand, to extract imaging parameters with highest accuracy, console screen frames containing exam card details can be processed at the highest resolution and sampled at a faster rate to track changes the local operator LO is making to the scan parameters. Similarly, to capture reconstructed images appearing on the screen in a robust fashion once the exam enters reformatting stage the quality and rate of console frames should increase.

Likewise, certain workflow steps during the medical imaging examination require more detailed information obtained from the medical imaging bay 3 video feed 17 than others. For instance, while the patient is being positioned by the local operator LO, video data 17 from the medical device 2 could allow the remote expert RE or even an AI algorithm to identify errors in patient positioning, safety concerns, etc. However, once the medical imaging examination is under way, typically very little information can be gleamed from medical imaging bay 3 video feed 17. Similarly, the patient may need to be observed more closely during contrast injection but when the medical device 2 is empty, or cleaning is taking place, a high-quality video feed 17 is unnecessary.

Some examples of approaches used for data management include 1) increasing or decreasing frame rate at which console screen data or medical imaging bay 3 video feed 17 are processed (i.e., processing 1 frame every second vs processing 1 frame every minute), 2) using low resolution data versus preserving high resolution content, 3) reducing image complexity by automatically cropping/blurring images, etc. In short, based on the situational context, minimal image quality requirements are identified, and streaming videos (either console screen or scanner room feed) are processed accordingly.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

In the foregoing detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

## Claims

1. A non-transitory computer readable medium (26s) storing instructions executable by at least one electronic processor (14s) to perform a method (100) of assisting a local operator (LO) during a medical procedure performed using a medical device (2), the method comprising:
acquiring a video (17) of at least one component of the medical device, wherein the video comprises a time sequence of image frames;
as the video is acquired, transmitting the acquired video to a remote electronic processing device (12) operable by a remote expert (RE);
as the video is acquired, determining a state of the medical procedure as a function of time from the acquired video; and
as the video is acquired, adjusting a data stream transmission setting of the transmitting as a function of time based on the determined state of the medical procedure as a function of time.

2. The non-transitory computer readable medium (26s) of claim 1, wherein the acquiring of the video (17) comprises:
screen-scraping a display of the medical device (2) wherein the time sequence of image frames comprises a time sequence of one of screen-scraped images of the display or images of the display provided by a video cable splitter (15).

3. The non-transitory computer readable medium (26s) of claim 1, wherein the acquiring of the video (17) comprises:
acquiring the video using a video camera (16) arranged to image a display of the medical device (2) wherein the time sequence of image frames comprises a time sequence of images of the display.

4. The non-transitory computer readable medium (26s) of any one of claims 1-3, wherein the video (17) comprises video of a controller display (10) of the medical imaging device, and the determining a state of the medical imaging device from the acquired video includes:
determining one of an idle state, a protocol prescription state, and an active scanning state of the medical imaging device.

5. The non-transitory computer readable medium (26s) of any one of claims 1-4, wherein the adjusting includes:
adjusting a transmitted frame rate of the acquired video (17) to the remote electronic processing device (12).

6. The non-transitory computer readable medium (26s) of any one of claims 1-5, wherein the adjusting includes:
adjusting a resolution of the image frames of the acquired video (17) transmitted to the remote electronic processing device (12).

7. The non-transitory computer readable medium (26s) of any one of claims 1-7, wherein the adjusting includes:
adjusting a compression parameter of a data compression process applied to the acquired video (17) before transmission to the remote electronic processing device (12).

8. The non-transitory computer readable medium (26s) of any one of claims 1-7, wherein the method (100) further includes:
providing a user interface (UI) (28) on a display device (24) of the remote electronic processing device (12), the UI including a user control button selectable by the SE to adjust the data stream transmission setting of the transmitting.

9. The non-transitory computer readable medium (26s) of any one of claims 1-7, wherein the method (100) further includes:
automatically detecting two-way communication between the local operator (LO) and the remote expert (RE); and
adjusting the data stream transmission setting of the transmitting as a function of time further based on the automatic detection of the two-way communication.

10. The non-transitory computer readable medium (26s) of any one of claims 1-9, wherein determining a state of the medical procedure from the acquired video (17) includes:
using a structural similarity index metric to determine the state of the medical procedure.

11. The non-transitory computer readable medium (26s) of any one of claims 1-9, wherein determining a state of the medical procedure from the acquired video (17) includes:
using text screen-scraped from the acquired video to determine the state of the medical procedure.

12. The non-transitory computer readable medium (26s) of any one of claims 1-11, wherein determining a state of the medical procedure from the acquired video (17) includes:
determining a state of a medical device (2) based at least in part on a medical procedure protocol; and
determining a time segment during the medical procedure protocol that the remote expert (RE) monitors the medical procedure; and
outputting an indication that the remote expert should be assisting during the medical procedure to the remote electronic processing device (12) operable by the remote expert.

13. A non-transitory computer readable medium (26s) storing instructions executable by at least one electronic processor (14s) to perform a method (100) of assisting a local operator (LO) during a medical procedure performed using a medical device (2), the method comprising:
transmitting an acquired video (17) of at least one component of the medical device to a remote electronic processing device (12) operable by a remote expert (RE), wherein the video comprises a time sequence of image frames;
determining a state of the medical procedure as a function of time from the acquired video; and
adjusting a data stream transmission setting of the transmitting as a function of time based on the determined state of the medical procedure as a function of time.

14. The non-transitory computer readable medium (26s) of claim 13, wherein the acquiring of the video (17) comprises:
screen-scraping a display of the medical device (2) wherein the time sequence of image frames comprises a time sequence of screen-scraped images of the display.

15. A method (100) of assisting a local operator (LO) during a medical procedure performed using a medical device (2), the method comprising:
transmitting an acquired video (17) of at least one component of the medical device to a remote electronic processing device (12) operable by a remote expert (RE), wherein the video comprises a time sequence of image frames;
determining a state of the medical procedure as a function of time from the acquired video;
adjusting a data stream transmission setting of the transmitting as a function of time based on the determined state of the medical procedure as a function of time;
automatically detecting two-way communication between the local operator (LO) and the remote expert (RE); and
adjusting the data stream transmission setting of the transmitting as a function of time further based on the automatic detection of the two-way communication.
